# EUROPEAN PATENT APPLICATION

(11) **EP 0 827 280 A2**
(43) Date of publication of application: **04.03.1998**
(21) Application number: 97306111.2
(22) Date of filing: 12.08.1997
(51) Int. Cl.: H03K 17/567, H03K 3/57, A61N 5/10

(54) **Pulse generator**

(30) Priority: 28.08.1996 US 704054
(71) Applicant: SIEMENS MEDICAL SYSTEMS, INC., Iselin, New Jersey 08830 (US)
(72) Inventor: Hitchcock, Roger, San Leandro, CA 94577 (US); Marziale, Michael, El Sobrante, CA 94803 (US); Thompson, Lance, San Leandro, CA 94579 (US)
(74) Representative: Fenlon, Christine Lesley

(57) **Abstract**

A high voltage pulse generating circuit comprising: a DC power source (102); a flyback transformer (120); a solid state switching circuit (110) connected between the output of the DC power source and the primary winding of the flyback transformer; primary winding current sensing means (121) for generating a signal indicative of the amplitude of the current flowing in the primary winding of the flyback transformer; and a controller (122) arranged to receive as an input the primary winding current signal from the primary winding current sensing means and to generate as an output to the switching circuit a control signal for decoupling the flyback transformer from the DC power source when the amplitude of the current in the primary winding reaches a desired level so as to initiate charging of a pulse forming network (176). A discharge switch (175) actuated by a further control signal from the controller (122) is then closed to discharge the pulse forming network across a pulse transformer (185).

## Description

The present invention relates to pulse generators and, more particularly, to pulse generating circuits which may be used as power supplies for linear accelerators, most especially linear accelerators of radiation treatment devices.

Radiation-emitting devices are used as radiation therapy devices for the treatment of patients. A radiation therapy device generally comprises a gantry which can be swivelled around a horizontal axis of rotation in the course of a therapeutic treatment. A linear accelerator is located in the gantry for generating a high energy radiation beam for therapy. This high energy radiation beam can be an electron or electromagnetic (e.g. X-ray) beam. During treatment, this radiation beam is trained on one zone of a patient lying in the isocentre of the gantry rotation.

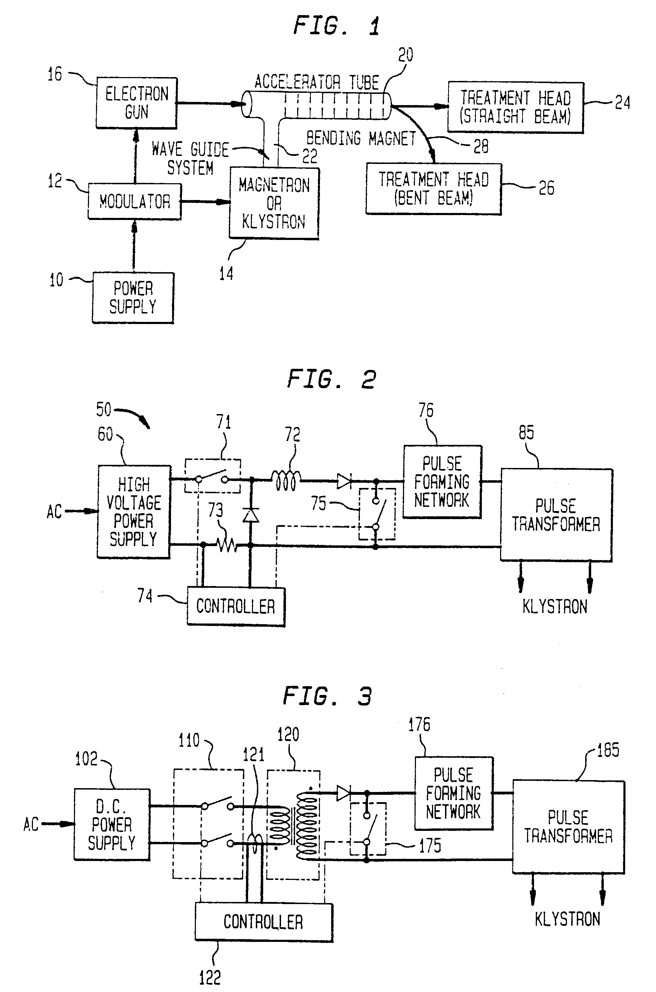

In this arrangement, radiation is generated by applying an electron beam to a target to generate X-rays. The electron beam is typically generated in a linear accelerator that is powered by a klystron-based power supply having a power output in the 10 to 30 kW range. Figure 1 of the accompanying drawings is a block diagram of a known medical linear accelerator and auxiliary components. A power supply 10 provides D.C. power to a modulator 12. The modulator 12 includes a pulse forming network and a switch tube known as a hydrogen thyratron. A thyratron is a low pressure gas device with a thermionic cathode. Over time, the cathode depletes itself, this depletion defining the operational life of the thyratron. The high voltage pulses from the modulator 12 are flat-topped D.C. pulses of a few microseconds in duration. These pulses are delivered to a magnetron or klystron 14 and simultaneously to an electron gun 16. Pulsed microwaves produced in the magnetron or klystron 14 are injected into an accelerator tube 20 via a waveguide system 22. Electrons produced by the electron gun 16 are pulse injected into the accelerator tube 20 with appropriate timing. High energy electrons emerge from the accelerator tube 20 in the form of a beam of approximately 3 mm in diameter. The electron beam can be directed to a treatment head 24 as a straight beam or to a treatment head 26 as a bent beam. If the electrons are sent to a treatment head 26, the electrons are bent by a bending magnet 28 through a suitable angle (e.g. 270 degrees) between the accelerator tube 20 and the target.

Prior art power supplies for linear accelerators tend to be large and heavy, and can significantly increase the cost and size of the medical treatment system. One prior art system utilizes a high voltage transformer/rectifier system to generate a 21 kV DC power source from a conventional three-phase 208 V power source. The high voltage DC source is then used to generate a 15 kV pulse that is converted to the required 150 kV pulse via a high voltage pulse transformer. The high voltage transformer/rectifier assembly typically weighs in the region of 225 kg (500 lbs) and occupies 0.2 cubic metres (8 cubic feet) and, as a result, is housed in a cabinet separate from the linear accelerator. In addition to the floor space needed to house the accelerator system, the cabinet requires power transmission lines to couple the klystron output to the linear accelerator which further contribute to the cost and complexity of the system. Finally, the sheer weight of the system results in significant shipping costs.

According to one aspect of the invention, there is provided a high voltage pulse generating circuit for powering a klystron magnetron and the like. In one embodiment, the pulse generating circuit is used in a radiation treatment device. The high voltage pulse generating circuit includes a source of D.C. power, a flyback transformer, a sensor and a solid state switching circuit. The source of D.C. power has positive and negative terminals. The flyback transformer has a primary winding and a secondary winding. The primary winding in the flyback transformer has first and second terminals for connection to the source of D.C. power. The sensor generates a signal indicating the amplitude of the current in the primary winding. The solid state switching circuit couples the source of D.C. power to the primary winding of the flyback transformer. The primary winding is coupled to the power source in response to a control signal, and decoupled from the power source when a predetermined level of current is detected in the primary winding.

According to a second aspect of the invention, there is provided a high voltage pulse generating circuit, comprising: a source of D.C. power having positive and negative terminals; a flyback transformer having a primary winding and a secondary winding, said primary winding having first and second terminals for connection to said source of D.C. power; means for generating a signal indicative of current flowing in said primary winding, said signal indicating the amplitude of said current; and a solid state switching circuit for coupling said source of D.C. power to said primary winding of said flyback transformer in response to a control signal and for decoupling said D.C. power source from said primary winding in response to said signal indicating that a predetermined level of current was flowing in said primary winding.

According to a third aspect of the invention, there is provided a pulse generating circuit in a radiation treatment device, comprising: a radiation source capable of generating a radiation beam having a variable radiation output; a DC power source having positive and negative terminals; a flyback transformer having a primary winding and a secondary winding, the primary winding having first and second terminals for connection to the DC power source; and means for generating a signal indicative of current flowing in the primary winding, the signal indicating the amplitude of the current; wherein the D.C. power, the flyback transformer and the means for generating a signal are used to supply power to the radiation source such that the radiation beam is generated.

Further aspects of the invention are exemplified by the attached claims.

For a better understanding of the invention, and to show how the same may be carried into effect, reference will now be made, by way of example, to the accompanying drawings, in which:-
Figure 1 is a block diagram of a medical linear accelerator showing major components and auxiliary systems;
Figure 2 is a block diagram of a prior art high voltage power supply;
Figure 3 is a block diagram of a high voltage power supply according to an embodiment of the invention;
Figure 4 is a block diagram of a power switch;
Figure 5 is a block diagram of a high voltage switch; and
Figure 6 is a schematic perspective view of the pulse forming network.

Figure 2 is a block diagram of a prior art power supply system 50 for powering a klystron. The power supply system 50 converts 208 volt, 3 phase power to 15 kV, 1200 amp pulses of approximately 5 µs duration. These pulses are stepped up to 150 kV by a pulse transformer 85 whose output drives the klystron. The 15 kV pulses are generated by a pulse generating circuit that is powered by a 21 kV D.C. source. The 21 kV D.C. source is typically a high voltage transformer and rectifier assembly 60. This D.C. power supply occupies in the region of 0.2 cubic metres (8 cubic feet) and weighs approximately 225 kg (500 lbs).

The high voltage pulse generating circuit typically consists of an inductor 72 which resonantly charges a pulse forming network 76. The final pulse amplitude that is applied to the klystron is adjusted by controlling the amount of time a high voltage charge switch 71 is closed. The system measures the current flowing through a resistor 73 and the voltage at the pulse forming network 76 to determine the timing of the switch opening. The connection to the pulse forming network has been omitted from the drawing. A controller 74 utilizes the current and voltage measurements to control the switch closure duration. The range of adjustment in the final pulse amplitude that can be obtained with the inductor design shown in Figure 2 is limited because only a portion of the energy of the final pulse is stored in inductor 72. The conversion of the 208 volt power to a 21 kV D.C. source requires a substantial number of high voltage components that must be operable at high power levels and which require high voltage insulation and pose safety problems.

Figure 3 is a block diagram of a high voltage power supply 100 according to an embodiment of the invention. The power supply 100 comprises a flyback transformer 120 to power a pulse forming network 176. The flyback transformer 120 is powered from a 300 volt D.C. power supply 102. (This is a significant voltage reduction from the 21 kV D.C. power supply used in the prior art system shown in Figure 2). A solid state switch 110 is used to control the output voltage from the flyback transformer 120. A controller 122 senses the current flowing in the primary coil or winding of the flyback transformer 120, as shown schematically in Figure 3 with the reference numeral 121. When the current reaches the desired level, the switch 110 is opened and the energy stored in the flyback transformer 120 is transferred to the pulse forming network 176. After the pulse forming network 176 is charged, a high voltage switch 175 is closed to discharge the pulse forming network 176 thereby transferring the energy stored in the pulse forming network 176 to the primary coil or winding of a pulse transformer 185. The operation of the pulse forming network 176 and the pulse transformer 185 are substantially the same as described above with reference to Figure 2.

The flyback transformer 120 stores substantially 100 percent of the energy that is later transferred to the klystron pulse. Hence, a greater range of control over the output pulse amplitude sent to the klystron is possible. The control of the pulse amplitude is also simplified in that it can be controlled by opening switch 110 in response to a predetermined current being sensed in the primary winding of the flyback transformer 120. The switch 110 is operable at 300 volts. (In contrast, the switch 71 shown in Figure 2 is operable at 21 kV). Hence, a significant saving in cost can be achieved, in addition to improved reliability and safety.

Further, since the flyback transformer 120 is driven by a relatively low voltage power source, problems associated with higher voltage power supplies can be avoided. The power supply 102 requires only approximately 7000 cubic centimetres (1/4 cubic foot) of space and weighs only about 2 kg (5 lbs). In addition, the relatively low operating voltage provides for increased safety and reliability.

In this embodiment, the switch 110 is implemented as a pair of insulated gate bipolar transistors (IGBTs). Other solid state switches may also be used.

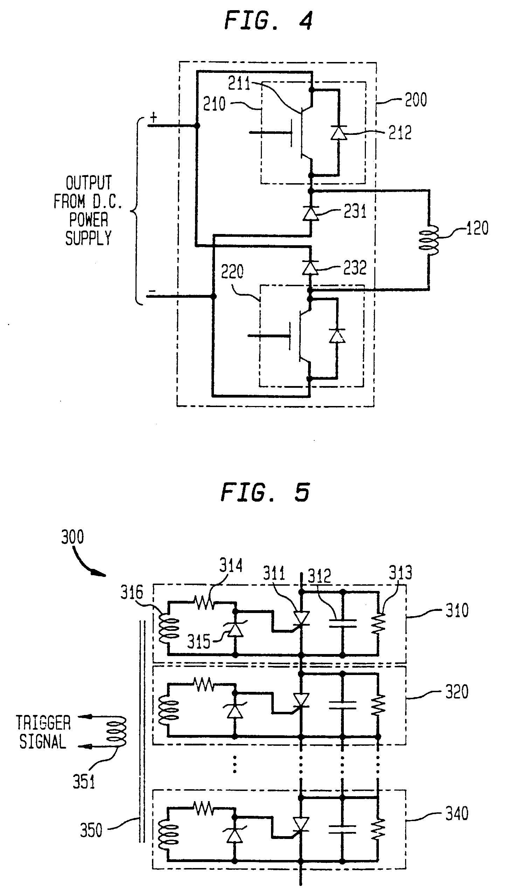

Figure 4 is a schematic drawing of a power switch 200. The power switch 200 utilizes two switching circuits shown at 210 and 220. Each switching circuit includes an IGBT 211 and a shunt diode 212. The switching circuits 210 and 220 are commercially available. The switching circuits 210 and 220 are arranged to connect the D.C. power supply to the primary winding of the flyback transformer 120. When the switching circuits 210 and 220 are in a state in which the primary winding of flyback transformer 120, is disconnected a reverse potential is generated across the primary winding. Shorting diodes 231 and 232 prevent this potential from damaging the switching circuits 210 and 220, respectively. The shorting diodes 231 and 232 redirect energy to the D.C. power supply where it is stored in the filter capacitors therein. As a result, the power is recovered for use in the next pulse.

The high voltage switch 300 (175 in Figure 3) is implemented as a high voltage semiconductor controlled rectifier (SCR) stack (i.e. a number of SCRs in series) as shown in Figure 5. (In prior art systems, such a switch is typically implemented with a gas thyratron which is generally less reliable and more costly than the SCR stack). The switch 300 is constructed from a number of SCR stages connected in series. The first, second, and last stages are shown at 310, 320 and 340, respectively. Each stage includes an SCR in parallel with a resistor and a capacitor, the resistor and capacitor being connected between the anode and cathode of the SCR. For example, stage 310 includes SCR 311, capacitor 312 and resistor 313. The capacitors and resistors are also connected in series to form a voltage divider network. The voltage divider assures that the same voltage is applied across each of the SCRs when the SCRs are not conducting. In the absence of the voltage divider, differences in the impedances of the SCRs in the non-conducting state can lead to different potentials being realized across each SCR when the SCR stack is not conducting. This can result in one of the SCRs being subjected to a potential difference in excess of its breakdown voltage.

The stack is triggered by coupling a signal through the inductor 316 in each stage. These inductors are the secondary stage of a pulse transformer 350, the signal being applied to the primary 351 of pulse transformer 350. Each stage includes a resistor and zener diode that ensures that the trigger voltage between the gate and cathode of the SCR in each stage are the same for each stage. The resistor and zener diode in the first stage are shown at 314 and 315, respectively.

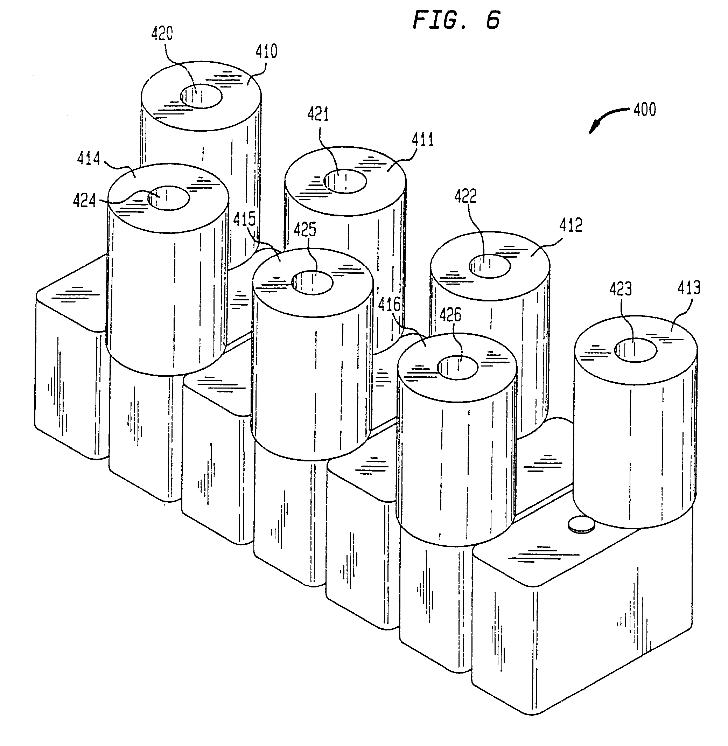

Figure 6 is a schematic perspective view of the pulse forming network 400 (176 in Figure 3) which includes inductors 410-416. Conventionally in a pulse forming network, a clip is placed on the inductors and the system must be shut down to manually change the inductance, the inductance being changed to fine tune the wave shape provided by the pulse forming network. This shutting down of the system and reviewing the wave shape is typically done repeatedly until the desired wave shape is obtained. A specially trained individual requires approximately 1 hour to fine tune the wave shape. In contrast, the present design uses aluminium slugs 420-426 which are placed inside the inductors 410-416. Each of the aluminium slugs 420-426 can be moved up and down, if desired while the system is running, to vary the inductance and fine tune the wave shape. The aluminium slugs 420-426 can be moved either manually or automatically. With this design, fine tuning takes approximately 3 minutes.

## Claims

1. A pulse generating circuit comprising:
a DC power source (102) ;
a flyback transformer (120); and
a switching circuit (110) connected between the output of the DC power source and the primary winding of the flyback transformer to selectively couple and decouple the flyback transformer and power source in response to a control signal, **characterised in that** the switching circuit (110) comprises a solid state switching element.

2. A circuit according to claim 1 and comprising:
primary winding current sensing means (121) for generating a signal indicative of the amplitude of the current flowing in the primary winding of the flyback transformer;

3. A circuit according to claim 2 and comprising:
a switching controller (122) arranged to receive as an input the primary winding current signal from the primary winding current sensing means and to generate as an output to the switching circuit said control signal, the control signal being generated in response to the primary winding current signal so as to decouple the flyback transformer from the DC power source when the amplitude of the current in the primary winding reaches a desired level.

4. A high voltage pulse generating circuit, comprising:
a DC power source (102) having positive and negative terminals;
a flyback transformer (120) having a primary winding and a secondary winding, said primary winding having first and second terminals for connection to said DC power source (102);
means (121) for generating a signal indicative of current flowing in said primary winding, said signal indicating the amplitude of said current; and
a solid state switching circuit (110) for coupling said DC power source (102) to said primary winding of said flyback transformer (120) in response to a control signal and for decoupling said DC power source from said primary winding in response to said signal indicating that a predetermined level of current was flowing in said primary winding.

5. A circuit according to claim 4 and comprising a switching controller (122) arranged to receive as an input said signal indicating that a predetermined level of current was flowing in said primary winding and to generate as an output to the solid state switching circuit (110) a decoupling signal to decouple the flyback transformer from the DC power source.

6. A circuit according to any one of the preceding claims, one terminal of the secondary winding of the flyback transformer (120) being coupled to the input of a pulse forming network (176), there being a discharge switch (175) arranged to selectively discharge the pulse forming network in response to a further control signal.

7. A circuit according to claim 6 when appended to claim 3 or 5, wherein the switch controller (122) is operable to generate as an output to the discharge switch (175) said further control signal, said further control signal being generated subsequent to said control signal so as to discharge the pulse forming network (176).

8. A circuit according to claim 7, wherein the discharge switch (175) includes a plurality of semiconductor controlled rectifier stages (310, 320... 340), each of the semiconductor controlled rectifier stages comprising a semiconductor controlled rectifier, a resistor and a control signal generator, the semiconductor controlled rectifier having an anode, a cathode and a gate, the semiconductor controlled rectifier being operable to conduct current from the anode to the cathode in the presence of a still further control signal generating a potential between the gate and the cathode, the still further control signal being generated by the control signal generator, the resistor being connected between the anode and cathode, and the stages being connected such that the semiconductor controlled rectifiers are connected in series.

9. A circuit according to claim 8, wherein the control signal generator in each said stage comprises a secondary winding of a pulse transformer, each said secondary winding being coupled to a common primary winding.

10. A circuit according to any one of claims 6 to 9, wherein the pulse forming network (176) comprises a plurality of inductors (410-416) and slugs (420-426) for varying the inductance of the pulse forming network.

11. A circuit according to claim 10, wherein the inductors have receiving portions for receiving respective ones of the slugs and there are means for adjusting the respective positions of the slugs relative to their receiving portions.

12. A circuit according to any one of the preceding claims, wherein the switching circuit (110) comprises as solid state switching elements first and second insulated gate bipolar transistors, the first insulated gate bipolar transistor coupling one terminal of the primary winding to the positive terminal of the DC power source when the first insulated gate bipolar transistor is in a conducting state and the second insulated gate bipolar transistor coupling the other terminal of the primary winding to the negative terminal of the DC power source when the second insulated gate bipolar transistor is in a conducting state.

13. A circuit according to any one of the preceding claims, wherein the switching circuit (110) comprises first and second diodes coupling the terminals of the primary winding respectively to the positive and negative terminals of the DC power source.

14. A circuit according to any one of the preceding claims, wherein the DC power source is operable to generate a voltage having a level of greater than 250 volts and less than 10 kV.

15. A circuit according to claim 14, wherein the DC power source is operable to vary the voltage level within the specified voltage range.

16. A sub-assembly for a radiation treatment device, the sub-assembly comprising a pulse generating circuit according to any one of the preceding claims.

17. A sub-assembly for a radiation treatment device, the sub-assembly having a pulse generating circuit comprising:
a DC power source (102);
a flyback transformer (120);
a switching circuit (110) connected between the output of the DC power source and the primary winding of the flyback transformer to selectively couple and decouple the flyback transformer and power source in response to a control signal; and
an output, coupled to the secondary windings of the flyback transformer, for supplying voltage pulses to a radiation source of a radiation treatment device.

18. A sub-assembly according to claim 17, wherein the switching circuit (110) comprises a solid state switching element.

19. A sub-assembly according to claim 16, 17 or 18 in combination with a radiation source connected to receive voltage pulses from the pulse generating circuit to generate a radiation beam.

20. A radiation treatment device comprising a pulse generating circuit according to any one of claims 1 to 15, a sub-assembly according to claim 16, 17 or 18, or a sub-assembly and radiation source according to claim 19.
